# EUROPEAN PATENT APPLICATION

(11) **EP 2 266 963 A1**
(43) Date of publication of application: **29.12.2010**
(21) Application number: 09305579.6
(22) Date of filing: 19.06.2009
(51) Int. Cl.: C07D 239/91, C12Q 1/37, G01N 33/533

(54) **Fluorogenic peptidase substrate**

(71) Applicant: ECOLE NORMALE SUPERIEURE DE LYON, 69364 Lyon Cedex 07 (FR); CENTRE NATIONAL DE LA RECHERCHE SCIENTIFIQUE, 75794 Paris Cedex 16 (FR)
(72) Inventor: Hasserodt, Jens, 69006 Lyon (FR); Zhang, Xiao-bing, Changsha Hunan (CN); Waibel, Michael, 69007 Lyon (FR)
(74) Representative: Sarlin, Laure V.

(57) **Abstract**

The invention is related to new peptidase substrates of formula (I) : 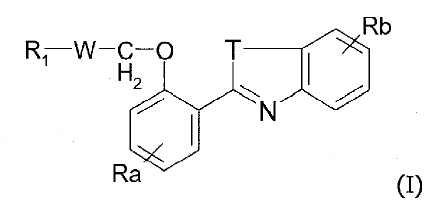 wherein
- R₁ is a peptidyl or amino acid moiety directly linked to W via its carbonyl function or one of its carbonyl functions, or, R₁ is a chain R'₁-NH-Y-C(0)- in which R'₁ is a peptidyl or amino acid moiety directly linked to the NH of the R₁ chain via its carbonyl function or one of its carbonyl functions and Y is an organic spacer,
- W is O, NH or NR₂ with R₂ representing a (C₁-C₄)alkyl,
- T and Rb are as defined in claim 1_{.}

and a process for detecting the presence of a catalytically active peptidase, using one of such substrates.

## Description

The invention relates to the technical field of probes for detecting enzyme activity. In particular the invention concerns new fluorogenic substrates for detecting the presence of a catalytically active peptidase and a detection process using such substrates.

In the analysis of a biological or chemical sample, the detection of a peptidase (or protease) activity may be very useful. Whole organisms, cells or cell extracts, biological fluids or chemical mixtures are examples of biological or chemical samples in which peptidase activity can be detected. Exo- and endoproteases are a vast family of enzymes and include numerous biomarkers of diverse pathologies. They are also implicated in many benign cellular processes and are thus the object of countless studies by cellular biologists. So, their detection can give information about a particular metabolic or disease state, for instance.

Accordingly, a probe capable of detecting peptidase activity is very useful. Detection of fluorescence emission is easy to implement, so fluorescent probes are promising. Among the large class of small-molecule fluorophores, 2-hydroxyphenylquinazolinone (HPQ) is exceptional in being perfectly insoluble in aqueous/physiological media while being strongly fluorescent in the solid state only. Up until now, it has been impossible to make use of HPQ in the design of a molecular probe that reports on the activity of an acyl hydrolase (either an esterase or a peptidase). The only activities for which an HPQ-based probe has yet been conceived (and commercialized) are those of phosphatases and glycosidases. The reason for this is found in the impossibility to create a stable HPQ-based probe with an acylated phenolic hydroxyl; the resulting product is prone to rapid spontaneous hydrolysis which of course liberates free, insoluble HPQ, and thus generates a false fluorescent signal ("background signal").

In this context, the purpose of the invention is to propose new peptidase substrates that are nonfluorescent but react with peptidases to yield a fluorescent small molecule such as HPQ.

More precisely, the invention relates to a peptidase substrate of formula
(1) : 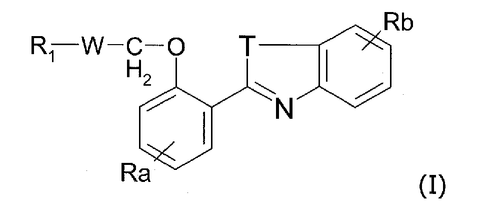 wherein
   - R₁ is a peptidyl or amino acid moiety directly linked to W via its carbonyl function or one of its carbonyl functions,
      or, R₁ is a chain R'₁-NH-Y-C(O)- in which R'₁ is a peptidyl or amino acid moiety directly linked to the NH of the R₁ chain via its carbonyl function or one of its carbonyl functions and Y is an organic spacer,
   - W is O, NH or NR₂ with R₂ representing a (C₁-C₄)alkyl,
   - T is -NH-C(O)- or -S-,
   - Ra and Rb, identical or different, are, each independently, hydrogen, Cl. Br, -OCH₃, -NO₂, -NRcRd with Rc and Rd, identical or different, representing a (C₁-C₄)alkyl.

The group R₁ is capable of being cleaved from the remainder of the substrate by action of the target peptidase resulting in an intermediate that immolates spontaneously and instantaneously to generate a fluorescent precipitate. The fluorescent precipitate obtainable from the peptidase substrate according to the invention, by cleavage of the covalent link between W and R₁, is another aspect of the invention. According to a particular embodiment, this precipitate is strongly fluorescent while the original peptidase substrate is hardly fluorescent or not fluorescent at all (off/on mode of action proper to the intact probe).

According to another aspect, the invention is related to a process for detecting the presence of a catalytically active peptidase, using a substrate according to the invention. More precisely, the invention relates to a process for detecting the presence of a catalytically active peptidase comprising the steps of :
- contacting a sample suspected of containing said peptidase with a substrate according to the invention;
- applying conditions suitable to allow formation of a fluorescent precipitate; and
- quantitatively or qualitatively analyzing said fluorescent precipitate.

In an embodiment of the invention, the analysis of the fluorescent precipitate comprises the steps of :
- exposing the fluorescent precipitate to a light source capable of producing light at a wavelength of absorption of the fluorescent precipitate; and
- detecting the resultant fluorescence of the precipitate.

This invention demonstrates the feasibility of employing fluorophores such as HPQ or HPQ analogues in the design of non-fluorescent probes susceptible to the activity of one of the members of the vast class of peptidases. This is achieved by incorporating the phenolic hydroxyl of the fluorophore into an acylated O/O acetal moiety rather than into a classic ester function. The acyl group will thus be part of an intelligent spacer, except for if it is the C terminus of an amino acyl group or a peptidyl group being an enzyme-recognizable entity directly grafted onto the O/O acetal. Once the target peptidase has liberated a free amino group at the terminus opposite the acyl group of the spacer, the latter spontaneously cyclises thereby releasing the O/O acetal of the fluorophore that, in return, immolates to give free, fluorescent fluorophore and formaldehyde. In the invention, the simplest fluorophore with Ra=Rb=H can be used. A more complex fluorophore with different substitutions can be used. For their preparation, it can be referred to EP 0641 351 or WO 2004/058787, for instance.

This invention thus makes the activity of peptidases accessible by fluorescence imaging using an HPQ (or HPQ analog)-based probe. Very low to zero background signal generation is observed. The probe itself is not fluorescent, especially not at the emission wavelength of free, solid HPQ, where the detection/imaging instrument is set. The probe thus functions in the off/on mode and can be used for the development of assays with maximal sensivity. This invention targets exo-(amino-)peptidases but should also be useful for the design of probes that detect endopeptidases with elevated selectivity for particular amino acid sequences. This should be possible by coupling a peptide sequence specific for a particular endoprotease to the terminus of the intelligent spacer. Additionally, decorating the spacer itself with amino acid side chains could contribute extra specificity to the process of enzyme recognition.

So, in an alternative embodiment of the invention, the substrate of formula (I) presents a R₁ group which is a chain R'₁-NH-Y-C(0)- in which R'₁ is a peptidyl or amino acid moiety directly linked to the NH of the R₁ chain via its carbonyl function or one of its carbonyl functions and Y is an organic spacer which represents -CR₃R'₃-(CR₄R'₄)ₙ -CR₅R'₅-CR₆R'₆- in which n is equal to 0 or 1 and R₃, R'₃, R₄, R'₄, R₅, R'₅, R₆ and R'₆, identical or different, are, each independently, a hydrogen atom, a (C₁-C₄)alkyl optionally substituted, an aryl group optionally substituted, or a lateral chain of a natural amino acid or R₃ and R'₃ and/or R₄ and R'₄ and/or R₅ and R'₅ and/or R₆ and R'₆ are linked together to form a (C₃-C₇)cycloalkyl.

In a preferred embodiment of such an organic spacer Y, R₃, R'₃, R₄, R'₄, R₅, R'₅, R₆ and R'₆, identical or different, are, each independently, a hydrogen atom, a methyl group or a lateral chain of a natural amino acid.

In a preferred embodiment of such an organic spacer Y, R₆ and R'₆ are identical and represent a hydrogen atom or a methyl group.

In a preferred embodiment of such an organic spacer Y, R₃ represents a hydrogen atom and R'₃ represents a lateral chain of a natural amino acid.

In a preferred embodiment of such an organic spacer Y, R₄, R'₄, R₅ and R'₅ are identical and represent a hydrogen atom.

In an alternative embodiment of the substrate according to the invention W=0.

In another alternative embodiment of the substrate according to the invention W=NCH₃.

In an alternative embodiment, the peptidase substrate according to the invention is of formula (IA) : 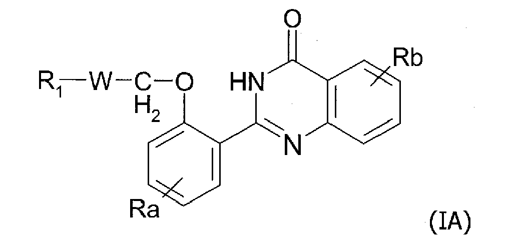 wherein R₁, W, Ra and Rb are as defined above for (I). According to a particular embodiment, Ra=Rb=H.

All possible peptidyl or amino acid moieties can be used in R₁ or R'₁ present in the peptidase substrate of formula (I) or (IA). The amino acids can be fonctionalized or not, especially in N-terminus of R₁ or R'₁. According to one embodiment of the invention, the peptidyl or amino acid residue has at most 10 amino acids in which the amino acids are identical or different. Preferably, for reasons of substrate costs, the peptidyl or amino acid residue has at most 4 amino acids in which the amino acids are identical or different. The amino acid residue or the amino acids of the peptidyl residue are preferably chosen from the natural proteinogenic amino acids. Nevertheless, the N-terminus of the amino acid residue or of the peptidyl residue can be fonctionalized with an acyl group -COR', R' being a (C₁-C₆)alkyl group or a O-(C₁C₆)alkyl group.

For instance, the following peptidyl moieties can be cited : Ser-Gln-Asn-Tyr- (the N-terminal portion of the preferred cleavage sequence of HIV-1 peptidase), or other peptidyl moieties P4-P3-P2-P1- presented in **Table 1,** in which the N-terminus of P₁ can be functionalized with an acyl group -COR', R' being a (C₁-C₆)alkyl group or O-(C₁C₆)alkyl group. The cleavage principle of these polyproteins is shown in **Scheme 1** hereafter.

**Table 1 Cleavage sites of the polyproteins Pr55^{gag} and Pr160^{gag-pol} that are selectively cleaved by HIV-1 peptidase.**

| Site | P4 | P3 | P2 | **P1** | **P1'** | P2' | P3' | P4' |
|---|---|---|---|---|---|---|---|---|
| MA/CA | Ser | Gln | Asn | **Tyr** | **Pro** | Ile | Val | Gln |
| CA/p2 | Ala | Arg | Val | **Leu** | **Ala** | Glu | Ala | Met |
| P2/NC | Ala | Thr | Ile | **Met** | **Met** | Gln | Arg | Gly |
| NC/p1 | Arg | Gln | Ala | **Asn** | **Phe** | Leu | Gly | Lys |
| p1/p6 | Pro | Gly | Asn | **Phe** | **Leu** | Gln | Ser | Arg |
| TF/PR | Ser | Phe | Asn | **Phe** | **Pro** | Gln | Ile | Thr |
| PR/RT | Thr | Leu | Asn | **Phe** | **Pro** | Ile | Ser | Pro |
| PT/IN | Arg | Lys | Val | **Leu** | **Phe** | Leu | Asp | Gly |
| RT (internal) | Alu | Glu | Thr | **Phe** | **Tyr** | Val | Asp | Gly |

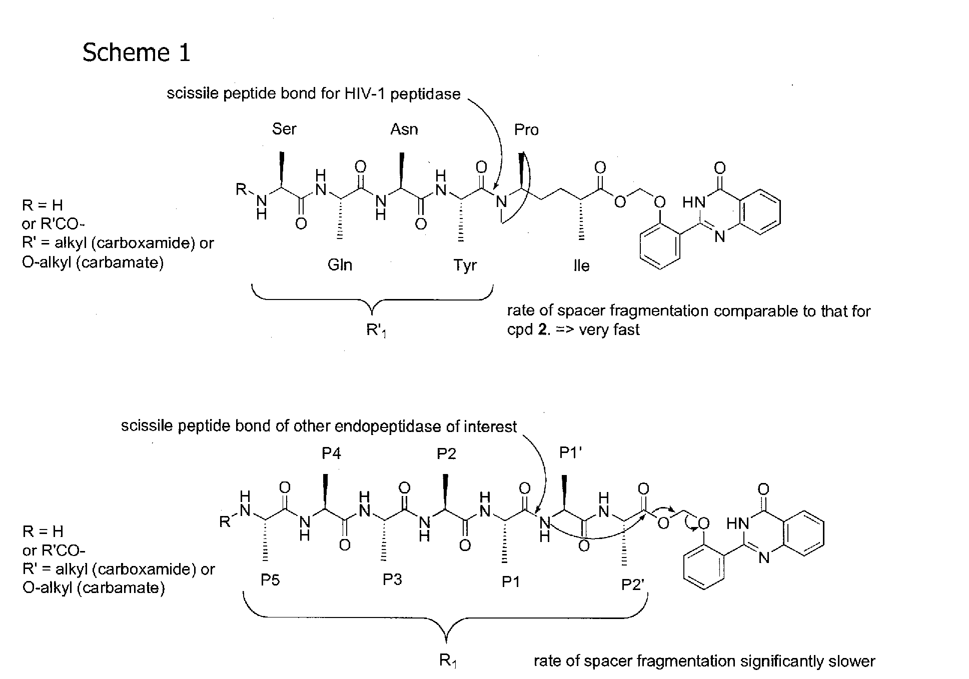

The nomenclature of amino acid residues used in Scheme 1 is that of Schechter and Berger (Schechter, I. ; Berger, A. Biochem. Biophys. Res. Commun. (1967), 27(2):157-162.)

The peptidyl or amino acid moiety is chosen for its adequacy with the known sequence selectivity of the targeted peptidase, which will recognize it. For instance, the peptidyl or amino acid moiety will be chosen for its adequacy with a peptidase implicated in certain diseases, listed in **Table 2.**

**Table 2 Examples of some peptidases implicated in certain diseases.**

| Peptidase | Class | function | Disease |
|---|---|---|---|
| HIV-1 peptidase | Asp | HIV replication | AIDS |
| Renin | Asp | Angiotensin I generation | Hypertension |
| Thrombin | Ser | Blood coagulation | Myocardial |
| | | | infarction |
| Tryptase | Ser | Phagocytosis | Asthma |
| Cathepsin K | Cys | Osseous resorption | Osteoporosis |
| ACE | Zinc | Angiotensin II generation | Hypertension |
| Plasmepsines I | Asp | Hemoglobin degradation | Malaria |
| and II | | | |
| β-Secretase | Asp | Amylioide β synthesis | Alzheimer's |
| | | | disease |
| Caspase | Cys | Apoptosis | Cancer |

The possible functionalization of the N terminus of a given probe with an acyl group (R'CO-) stems from the fact that some endoproteases will not interact with a substrate with a free amino group at this terminus. Solid-phase peptide synthesis (SPPS) using carbamate protecting groups often makes it more straightforward to synthesize a probe displaying a carbamate at this N terminus.

Some terms used in the definition of substrate (I) are defined hereafter.

By alkyl is meant a saturated hydrocarbon chain which can be linear or branched. Methyl, ethyl, n-propyl, isopropyl, iso-butyl, tert-butyl, n-pentyl and n-hexyl, are examples of a (C₁-C₆)alkyl group (alkyl with 1 to 6 atoms of carbon).

By aryl is meant a phenyl, naphthyl, or cinnamyl group.

When the group alkyl or aryl is substituted, it can be substituted by one or several substituants chosen among Cl, Br, -NO₂, -OCH₃, -CONH(C₁-C₆)alkyl), -CONH(aryl), -NHCO(C₁-C₆)alkyl, -NHCO(aryl), -S0₃H, - NH₂, -NH(C₁-C₆)alkyl, -N[(C₁₋C₆)alkyl]₂.

By (C₃-C₇)cycloalkyl is meant a saturated hydrocarbon chain of 3 to 7 carbons which forms a cycle. cyclopropyl, cyclopentyl, cyclohexyl and cycloheptyl are examples of a (C₃-C₇)cycloalkyl group.

"Fluorescence" is the property by which a molecule that is excited by light of a given wavelength emits light at a longer wavelength. Fluorescence is a phenomenon that results from the interaction of a fluorophore with an incident photon. This process is referred to as excitation. Absorption of the photon causes an electron in the fluorophore to rise from its ground state to a higher energy level. Then, the electron reverts to its original level, releasing a photon. This process is referred to as fluorescence emission. The fluorophore then emits light at a longer wavelength than that of the absorbed photon. This is simply because the energy of the emitted photon is lower than that of the absorbed photon, due to energy dissipation during the excited state lifetime. This definition is given in WO 2004/058787.

The compounds (I) according to the invention are called "peptidase substrate" because they are transformed into another substance during a chemical reaction catalyzed by a peptidase. During such a reaction, the compounds (I) (named also "probe") are cleaved into a fluorescent precipitate and a non-fluorescent product upon action of the specific peptidase.

The substrates of the present invention have good cell membrane permeability compared to other known fluorogenic enzyme substrates and will be able to easily enter cells, and therefore these substrates will be used for various applications in a wide variety of cells. In addition, the substrates according to the present invention are generally soluble but very little fluorescent in water, however, they release a highly fluorescent precipitate in an aqueous solution containing the substrate and the corresponding peptidase. Conditions suitable to allow formation of a fluorescent precipitate upon peptidase hydrolysis are purely aqueous media, such as a buffered medium or a physiological medium. Importantly the precipitate is formed upon peptidase hydrolysis without compromising the peptidase activity. So, their use for localization studies in biological specimens or detection of discrete bands in non-denaturing PAGE and in Western blotting is possible. More details about the conditions and the techniques of detection that can be used, are given in WO 2004/058787 and EP 0641351 which can directly be applied to the present invention.

So, the peptidase substrates according to the invention have a lot of potential applications. Examples of such applications include:
(a) design of bacterial colony assays. These are usually conducted on an agar plate (Petri dish) where up to 3000 colonies can be distinguished without having to separate them actively into separate compartments such as wells contained in a multi-well plate. It is thus possible to perform massive parallel testing of a protein library of one's own making expressed by a classic (often commercial) bacterial host. This collection of proteins can of course contain one of particular interest, say a protease with selectivity for a specific amino acid sequence, or a protease hydrolyzing a non natural carboxamide bond. In the field of Directed Evolution of proteins in general or enzymes in particular, there is great demand for efficient and sensitive assays to screen enormous numbers of protein variants easily exceeding 10⁶. The application of the probe according to the invention can most easily be envisaged by dissolving it in the agar solution before it is being poured into the plate where it gellifies. Alternatively, substrates are incubated with the colonies by soaking a filter before pressing it onto the colonies. The principal advantage that the probe according to the invention contributes to such a colony assay is the on-site precipitation of the fluorophore; a dilution of the fluorescent signal by diffusion is thus much reduced, permitting longer incubation times and thus higher sensitivity for the assay. The extremely large Stokes shift of HPQ (approx. 140 nm) or any HPQ analog should not remain unappraised : it also contributes to excellent sensitivity and makes it easily distinguishable from native fluorescence issued from the biological sample.
(b) in vitro (histology) and in vivo imaging. In view of the very low solublity of free HPQ or any HPQ analog, any release of it in a complex biological environment allows for the fluorescence imaging with high spatial control. Fluorescence imaging is a widely applied craft to highlight subcellular structures. Localization of a specific peptidase activity with high resolution may be possible by use of probe according to the invention.

By contrast, passive fluorescent probes (those that are not transformed by an enzyme) are equipped with a ligand specific for a cellular receptor in order to lock onto this receptor by non-covalent interactions; they are thus not safe from signal dilution effects. On top of this, one receptor equals at best one fluorescent label. The sensivity of an assay based on such probes is necessarily much lower than that of an active probe that benefits from catalytic signal amplification.

The probe according to the invention may also serve macroscopic fluorescence imaging, i.e. on the level of a whole organism. Should the probe penetrate the cell wall in order to reach the peptidase activity of interest, liberation of the free fluorophore rules out swift signal dilution by partitioning with the extracellular space. Generally, this allows for extending incubation times before imaging, especially useful when the enzyme activity is weakly expressed.

The peptidase substrates according to the invention can be prepared according to known techniques, detailed in the following examples. For instance, an optionally protected peptidyl or amino acid moiety can be grafted onto the spacer group. After a coupling step to introduce the fluorophore via its phenolic hydroxyl, the peptidyl or amino acid moiety may be deprotected. The complete probe thus obtained can be purified, before utilization, by conventional techniques.

The following examples, which refer to the annexed figures, illustrate the invention.
**Figure 1** represents the emission spectra of substrate **2** before and after incubation with leucyl aminopeptidase.
**Figure 2** represents the evolution of fluorescence with time of substrates **1** and **2** with and without leucyl aminopeptidase.
**Figure 3** represents the evolution of fluorescence with time of LB (Lysogeny broth) medium alone, LB medium with substrates **2,** LB medium with leucyl aminopeptidase and LB medium with substrates **2** and with leucyl aminopeptidase.
**Figure 4** represents the evolution of fluorescence with time of LB medium with leucyl aminopeptidase, with or without substrates **2** and/or with and without an inhibitor leucyl aminopeptidase..
**Figure 5** represents the evolution of fluorescence with time of LB alone and LB with or without bacterial cell line and with or without substrates **2.**

### GENERAL

Column chromatography was performed using silicagel Si 60 (40-63 µm). ¹H and ¹³C NMR spectra were recorded at 200.13 MHz, 50.13 MHz, respectively. Chemical shifts (δ) are reported in ppm (s = singlet, d= doublet, t = triplet, m = multiplet, br = broad) and quoted with reference to tetramethyl silane or from solvent signals. NMR coupling constants (J) are reported in Hertz. Fluorescence assays were carried out in black roundbottom polypropylene 96-well-plates (NUNCLONE, Nunc Inc.) and recorded on a microplate spectrofluorimeter (Gemini XPS by Molecular Devices).

Except when specified, chemicals were purchased in analytical reagent grade and used without further purification. 3,3-Dimethylpyrrolidin-2-one **(7)** was synthesized following a reported procedure [Org. Lett. 2001, 3, 751-754]. Toluene, DMF, triethylamine, CH₂Cl₂, and acetonitrile were distilled from calcium hydride and stored over molecular sieves. Tetrahydrofuran (THF) was freshly distilled from sodium chips under argon with benzophenone as indicator. Twice distilled water was used throughout all experiments.

The following abbreviations are used :
Me = methyl
Cbz = carbobenzoxy
tBu = tertiary butyl
EDC = 1-ethyl-3-(3-dimethylaminopropyl) carbodiimide hydrochloride
TLC = thin layer chromatography
DMF = dimethylformamide
DCM = dichloromethane
DDQ = 2,3-dichloro-5,6-dicyano-1,4-benzoquinone
Substrates **1** and **2** are prepared as described on **Scheme 2** hereafter.

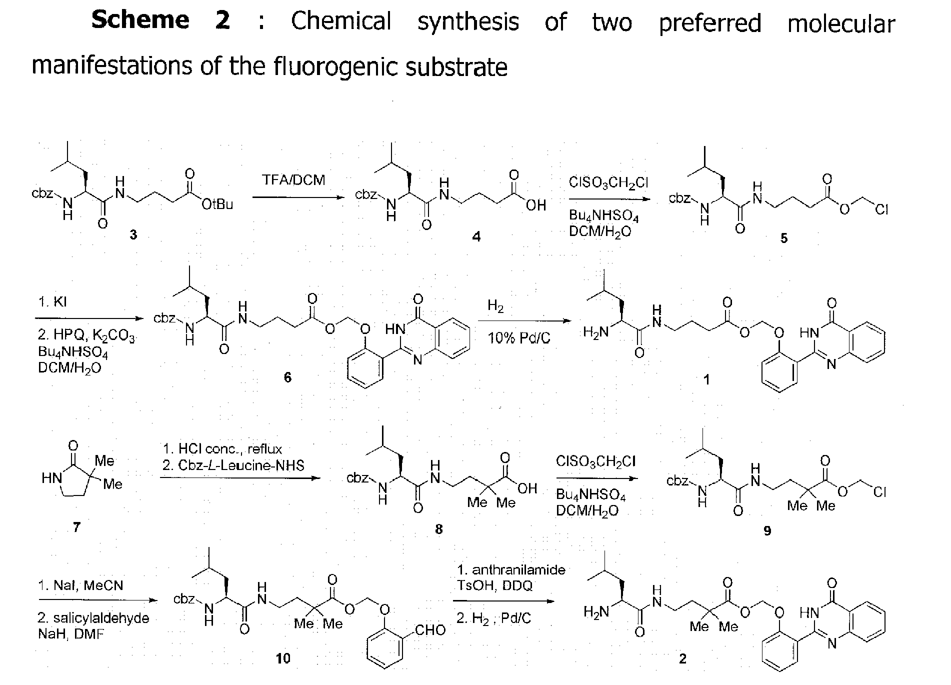

### EXAMPLE 1

**a) Preparation of *t*-butyl ester 3.** A stirred DMF solution (5 mL) of N-Cbz-L-leucine (265 mg, 1 mmol) in a 50 mL 3-necked flask was treated
with the hydrochloride of γ-aminobutyric acid *t*-butyl ester (195.6 mg, 1 mmol) EDC (192 mg, 1 mmol) and HOBt (135 mg, 1 mmol). Stirring was continued overnight. The mixture was extracted with CH₂Cl₂. The combined organic phases were washed first with NaHCO₃ and then with 1N HCl, and subsequently dried over anhydrous MgSO₄, filtered, and evaporated to dryness. The crude product was purified by silicagel column chromatography (CH₂Cl₂/ ethyl acetate, 10:1, v/v) to give **3** (394 mg, 97%) as a colorless solid. TLC : R_{f} = 0.28 CCH₂Cl₂/ ethyl acetate, 10: 1, v/v). ¹H NMR (200 MHz, CD₃CN) δ 0.80-0.86 (d, *J* = 6.1 Hz, 6H), 1.34 (s, 9H), 1.45-1.62 (m, 5H), 2.10 (t, *J* = 7.4 Hz, 2H), 3.02-3.12 (m, 2H), 3.88-3.99 (m, 1H), 4.99 (s, 2H), 5.72 (br, 1H), 6.63 (br, 1H), 7.29 (s, 5H); ¹³C NMR (50 MHz, CD₃CN) δ 20.71, 22.17, 24.56, 27.16, 32.14, 37.99, 40.96, 53.67, 65.99, 79.59, 127.54, 127.77, 128.33, 137.10, 156.02, 172.23; MS (ESI, positive mode): m/z 407.2 [M+H]⁺, 429.2 [M+Na]⁺.

**b) Preparation of chloromethyl ester 5:** A solution of **3** (394 mg) in CH₂Cl₂ (8 mL) and trifluoroacetic acid (TFA, 2 mL) was stirred at room temperature for 4 h, before being concentrated under vacuum. The residue was redissolved in CH₂Cl₂ (20 mL) and washed with water (3 x 20 mL). The organic layer was dried over anhydrous MgSO₄, filtered, and evaporated to dryness to give crude **4** as a colorless solid (320 mg, 0.91 mmol, 94 %). This material was directly introduced into the next step by treating it with a solution of sodium bicarbonate (1.0 g) in 8 mL of water. After stirring for 5 min, 8 mL of CH₂Cl₂ and 100 mg of tetrabutylammonium hydrogen sulfate were added. Subsequently, chloromethyl chlorosulfate (190 mg) dissolved in CH₂Cl₂ (3 mL) was slowly added to the mixture. After an additional 30 min of stirring, the organic phase was separated, washed twice with water, dried over anhydrous Na₂SO₄, filtered, and evaporated to dryness. After purification by silicagel column chromatography (CH₂Cl₂/ethyl acetate 10:1, v/v), **5** was obtained as a colorless solid (310 mg, 80% ). TLC : R_{f} = 0.3 (CH₂Cl₂/ ethyl acetate, 10:1, v/v). ¹H NMR (200 MHz, CD₃CN) δ 0.89 (d, *J* = 4.3Hz, 6H), 1.52-1.76 (m, 5H), 2.38 (t, *J* = 7.1 Hz, 2H), 3.12-3.21 (m, 2H), 3.99-4.02 (m, 1H), 5.07 (s, 2H), 5.73 (s, 2H), 5.81 (br, 1H), 6.72 (br, 1H), 7.36 (s, 5H). ¹³C NMR (50 MHz, CD₃CN) δ 20.68, 22.17, 24.02, 30.58, 37.68, 40.87, 53.68, 65.99, 69.03, 127.54, 127.77, 128.33, 136.95, 156.05, 171.25, 172.34. MS (ESI, positive mode): m/z 399.2, [M+H]⁺.

**c) Preparation of Cbz-protected target 6** : A mixture of chloromethyl ester **5** (310 mg, 0.78 mmol), potassium iodide (670 mg, 4.0 mmol) and dry acetonitrile (10 mL) was stirred at 50 °C for 10 h. The resulting salts were filtered off and washed with acetonitrile (10 mL) to obtain an acetonitrile solution of iodo derivative of **5.** It was evaporated to dryness and introduced directly into the next step.

A mixture of HPQ (192 mg, 0.8 mmol) and excess K₂CO₃ in water (15 mL) was allowed to stir for several minutes until HPQ was completely dissolved. Now, tetrabutylammonium hydrogen sulfate (0.8 mmol) and CH₂Cl₂ (10 mL) were added. After several minutes of stirring, a solution of the above iodo derivative of **5** (0.77 mmol) in CH₂Cl₂ (10 mL) was slowly added to the reaction mixture. The resulting biphasic system was vigorously stirred overnight. The phases were now separated and the aqueous layer was extracted with methylene chloride. The organic phases were combined and concentrated under vacuum to give an oily residue. After purification by silicagel column chromatography (CH₂Cl₂/ ethyl acetate, 10:8, v/v), **6** was obtained as an oil (130 mg, 27 %). TLC : R_{f} = 0.32 (CH₂Cl₂/ethyl acetate, 10:8, v/v). ¹H NMR (200 MHz, DMSO-d₆) δ 0.85 (d, *J* = 6.3 Hz, 6H), 1.57-1.64 (m, 5H), 2.31 (t, *J* = 7.8 Hz, 2H), 2.97-3.06 (m, 2H), 3.89-4.00 (m, 1H), 5.00 (s, 2H), 5.80 (s, 2H), 7.19-7.26 (m, 2H), 7.33 (s, 5H), 7.51-7.71 (m, 5H), 8.14 (d, 1H), 12.18 (s, 1H) ¹³C NMR (50 MHz, DMSO-d₆) δ 21.83, 23.25, 24.55, 30.96, 37.88, 53.51, 65.66, 86.36, 115.76, 121.38, 123.05, 124.87, 126.13, 127.05, 127.97, 128.07, 128.62, 134.79, 137.39, 149.23, 152.30, 154.78, 156.23, 161.58, 172.04, 172.59. MS (ESI, positive mode): m/z 601.2, [M+H]⁺.

**d) Preparation of substrate 1** : Compound **6** (130 mg, 0.22 mmol) was dissolved in MeOH (10 mL), and 10 % Pd-C (100 mg) was added. The mixture was stirred under a hydrogen atmosphere (1 bar) at room temperature for 1 h, then the catalyst was filtered off and the filtrate was evaporated to dryness. Purification by silicagel column chromatography (CH₂Cl₂/MeOH, 10:1, v/v) gave **1** (80 mg, 79 %) as a colorless oil. TLC: R_{f} = 0.39 (CH₂Cl₂/MeOH, 10:1, v/v). ¹H NMR (200 MHz, CDCl₃) δ 0.90 (d, *J* = 5.9 Hz, 6H), 1.66-1.83 (m, 5H), 2.36-2.42 (m, 2H), 3.23-3.30 (m, 2H), 3.67 (s, 1H), 5.93 (s, 2H), 7.07-7.21 (m, 2H), 7.44-7.50 (m, 2H), 7.76-77.0 (m, 2H), 8.27-8.31 (m, 2H); ¹³C NMR (50 MHz, CDCl₃) δ 21.40, 23.41, 24.89, 31.17, 37.93, 44.05, 53.54, 85.56, 115.00, 119.06, 123.79, 126.82, 127.92, 131.79, 133.00, 134.57, 149.29, 150.55, 150.84, 154.43, 172.06, 174.86; MS (ESI, positive mode): m/z 467.2, [M+H]⁺.

### EXAMPLE 2

### a) Preparation of compound 8

**2,2-Dimethyl-4-aminobutanoic acid** (see Chem. Eur. J. 2002, 8, 573-584 for this procedure applied to a similar compound) : 3, 3-Dimethylpyrrolidin-2-one **7** (300 mg, 2.7 mmol) was dissolved in 6 N HCl (20 mL) and refluxed for 12 hours. After evaporation of the volatile components, the remaining residue was co-evaporated with acetone. The crude hydrochloride of 2, 2-dimethyl-4-aminobutanoic acid (400 mg) was obtained as a slightly yellow solid and introduced directly into the next step.

For the following protocol applied to a different compound see J. Am. Chem. Soc. 2004, 126, 16368-16378 :
Cbz-*L*-leucine (530 mg, 2 mmol) and N-hydroxysuccinimide (230 mg, 2 mmol) were dissolved in dry acetonitrile (20 mL) and stirred for 15 min at 0 °C. The solution was treated with dicyclohexylcarbodiimide (454 mg, 2.2 mmol) and stirred at room temperature for one night. The urea precipitate was filtered off and the filtrate was concentrated to dryness. The resulting NHS ester of Cbz-*L*-leucine (Cbz-*L*-Leucine-NHS) was collected and used in the following step without further purification.

To a stirred solution of the crude hydrochloride of 2,2-dimethyl-4-aminobutanoic acid (400 mg, excess) in dry CH₂Cl₂/MeOH 1:1 (20 mL) and at 0°C was added a solution of Cbz-*L*-Leucine-NHS (2 mmol) in dry CH₂Cl₂ (5 mL). After 10 min, dry triethylamine (200 µL) was added, and stirring was continued overnight at room temperature. The phases were separated, the aqueous phase extracted with CH₂Cl₂ (3 x30 mL), the combined organic phases dried over anhydrous Na₂SO₄, before the solvent was removed under reduced pressure. Purification by silicagel column chromatography (CH₂Cl₂/MeOH, 10:1, v/v) gave **8** as a colorless oil (430 mg, 57%). TLC : R_{f} = 0.46 (CH₂Cl₂/MeOH, 10:1, v/v). ¹H NMR (200 MHz, CDCl₃) δ 0.90 (d, *J* = 3.9 Hz, 6H), 1.21 (s, 6H), 1.41-1.78 (m, 5H), 3.25-3.36 (m, 2H), 4.11-4.22 (m, 1H), 5.13 (s, 1H), 5.47 (br, 1H), 6.50 (br s, 1H), 7.33 (s, 5H); ¹³C NMR (50 MHz, CDCl₃) δ 21.91, 22.94, 24.74, 36.21, 40.73, 41.33, 53.74, 67.38, 128.10, 128.57, 136.00, 156.92, 172.39, 181.64; MS (ESI, positive mode): m/z 379.2 [M+H]⁺, 410.2 [M+MeOH]⁺.

**b) Preparation of chloromethyl ester 9.** To a stirred solution of sodium bicarbonate (1.0 g) in 8 mL of water was added 430 mg (1.14 mmol) of **8**. After 5 min, 8 mL of CH₂Cl₂ and 100 mg of tetrabutylammonium hydrogen sulfate were added. Subsequently, chloromethyl chlorosulfate (190 mg) dissolved in CH₂Cl₂ (3 mL) was slowly added to the mixture. After an additional 30 min of stirring, the organic phase was separated, washed twice with water, dried over anhydrous Na₂SO₄, filtered, and evaporated to dryness. The crude oil was purified by silicagel column chromatography (CH₂Cl₂/ethyl acetate 10:1, v/v) to give **9** as an oil (401 mg, 83%). TLC : R_{f} = 0.54 (CH₂Cl₂/ethyl acetate, 10:1, v/v). ¹H NMR (200 MHz, CDCl₃) δ 0.92 (d, *J* = 5.3 Hz, 6H), 1.23 (s, 6H), 1.64-1.81 (m, 5H), 3.21-3.31 (m, 2H), 4.06-4.16 (m, 1H), 5.10 (s, 2H), 5.71 (s, 2H), 6.10 (br s, 1H), 7.35 (s, 5H); ¹³C NMR (50 MHz, CDCl₃) δ 21.96, 22.99, 24.73, 24.89, 26.95, 35.93, 39.05, 41.28, 53.45, 67.18, 68.99, 128.11, 128.29, 128.60, 136.16, 156.23, 172.04, 175.58; MS (ESI, positive mode): m/z 427.2 [M+H]⁺, 458.2 [M+MeOH]⁺.

**c) Preparation of aldehyde 10:** Compound **9** (112 mg, 0.26 mmol) was converted to the iodo derivative following the same procedure as for compound 5 and used without purification in the next step.

NaH (20 mg, 60% suspension in oil, 0.5 mmol) was added to a solution of salicylaldehyde (61 mg, 0.5 mmol) in dry DMF (5 mL) and stirred at room temperature for 30 min. The solid was filtered off and the filtrate cooled to - 20 °C before being added to a precooled (-20 °C) solution of the iodo derivative of **9** in anhydrous DMF (5 mL). The temperature of the reaction mixture was slowly raised to room temperature, and stirring was continued for 2 h. The resulting mixture was poured into ice water and extracted with CH₂Cl₂ (3 x 30 mL). The solvents were evaporated and the residue purified to homogeneity by silicagel column chromatography (CH₂Cl₂/ethyl acetate 10:2, v/v) to give **10** as a colorless oil (64 mg, 48%). TLC : R_{f} = 0.51 (CH₂Cl₂/ ethyl acetate, 10:2, v/v). ¹H NMR (200 MHz, CDCl₃) δ 0.92 (d, *J* = 5.5 Hz, 6H), 1.2 (s, 6H), 1.50-1.77 (m, 5H), 3.15-3.26 (m, 2H), 4.04-4.15 (m, 1H), 5.11(s, 2H), 5.89 (s, 2H), 6.03 (br s, 1H), 7.17 (d, *J* = 7.8Hz, 2H), 7.34 (s, 5H), 7.59 (t, *J* = 7.8 Hz, 1H), 7.86 (d, *J* = 7.7 Hz, 1H), 10.44 (s, 1H); ¹³C NMR (50 MHz, CDCl₃) δ 22.98, 24.73, 25.00, 26.95, 36.00, 39.12, 41.46, 53.44, 67.17, 85.73, 114.98, 123.13, 128.10, 128.29, 128.59, 128.72, 135.93, 152.69, 158.93, 171.94, 175.58, 189.46; MS (ESI, positive mode) : m/z 513.2 [M+H]⁺.

**d) Preparation of substrate 2:** Aldehyde **10** (64 mg, 0.13 mmol) and anthranilamide (19 mg, 0.14 mmol) were dissolved in anhydrous methanol (30 mL) and stirred at room temperature for 2 h. *p*-Toluenesulfonic acid (2 mg) was added, and the mixture was refluxed for another hour. The resulting solution was cooled to 0°C, and DDQ (2,3-dichloro-4,5-dicyano-1, 4-benzoquinone, 30 mg, 0.13 mmol) was added in 3 portions over 30 min. The mixture was slowly brought to room temperature and stirred for further 2 h, before being evaporated under vacuum to give a brown solid. The solid was washed with cold benzene (2 x 50 mL) and purified by silicagel column chromatography (CH₂Cl₂/ethyl acetate, 10:5, v/v) to give the cbz-protected version of 2 as a brown oil (71 mg, 87%). TLC : R_{f} = 0.42 (CH₂Cl₂/ethyl acetate, 10:5, v/v); ¹H NMR (200 MHz, CDCl₃) δ 0.91 (d, *J* = 5.2 Hz, 6H), 1.19 (s, 6H), 1.48-1.59 (m, 3H), 1.70-1.74 (m, 2H), 3.10-3.21 (m, 2H), 4.10-4.18 (m, 1H), 5.07 (s, 2H), 5. 61 (br s, 1H), 5.93 (s, 2H), 6.65 (br s, 1H), 7.12-7.18 (m, 2H), 7.30 (s, 5H), 7.46-7.57 (m, 2H), 7.75-7.81 (m, 2H), 8.29-8.33 (m, 2H), 10.80 (s, 1H); ¹³C NMR (50 MHz, CDCl₃) δ 23.03, 24.74, 25.01, 36.03, 39.22, 41.54, 41.85, 53.67, 66.91, 86.15, 114.95, 123.76, 126.55, 126.94, 127.96, 128.10, 128.49, 131.82, 133.04, 134.70, 149.27, 150.46, 154.20, 156.27, 162.02, 172.35, 176.25; MS (ESI, positive mode): m/z 629.2 [M+H]⁺.

The cbz-protected version of **2** (71 mg, 0.11 mmol) was deprotected in the same manner as **1** was obtained from **6.** After purification by silicagel column chromatography (CH₂Cl₂/MeOH 10:1, v/v), **2** was obtained as a colorless oil (42 mg, 75% ). TLC : R_{f} = 0.39 (CH₂Cl₂/MeOH, 10:1, v/v). ¹H NMR (200 MHz, CDCl₃) δ 0.90 (d, *J* = 5.9 Hz, 6H), 1.20 (s, 6H), 1.60-1.76 (m, 5H), 3.13-3.23 (m, 2H), 3.27-3.33 (m, 1H), 5.96 (s, 2H), 7.18-7.29 (m, 2H), 7.48-7.49 (m, 2H), 7.77-7.78 (m, 2H), 8.28-8.33 (m, 2H); ¹³C NMR (50 MHz, CDCl₃) δ 23.42, 24.97, 35.46, 39.44, 41.48, 44.12, 53.56, 86.00, 114.96, 123.77, 126.49, 126.81, 127.90, 131.83, 133.02, 134.57, 149.34, 150.84, 154.42, 164.35, 171.48, 176.36; MS (ESI, positive mode): m/z 495.2 [M+H]⁺.

### EXAMPLE 3 : Detection of the presence of a catalytically active peptidase

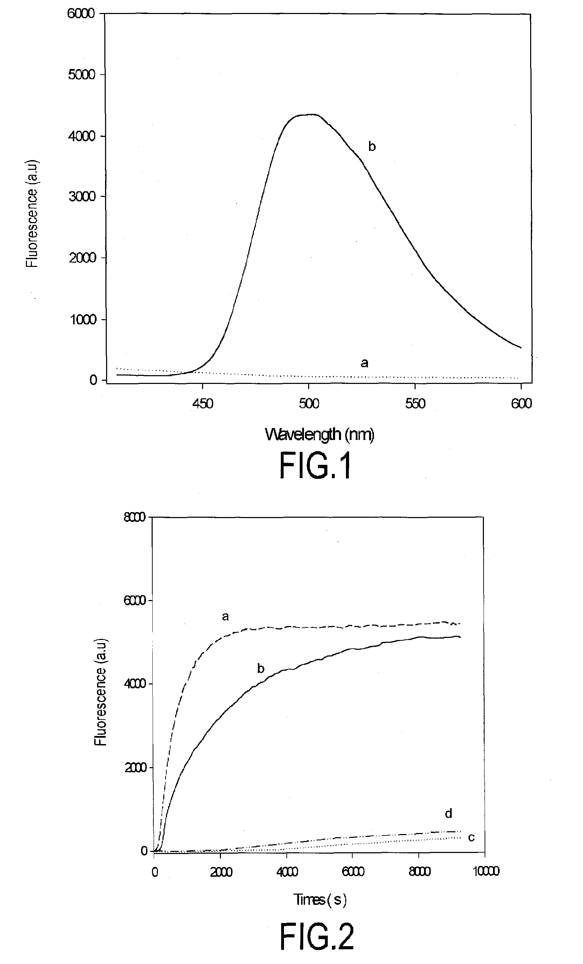

**Scheme 3** presents the reaction cascade initiated by cleavage of the substrate 1 or 2 by a target peptidase. 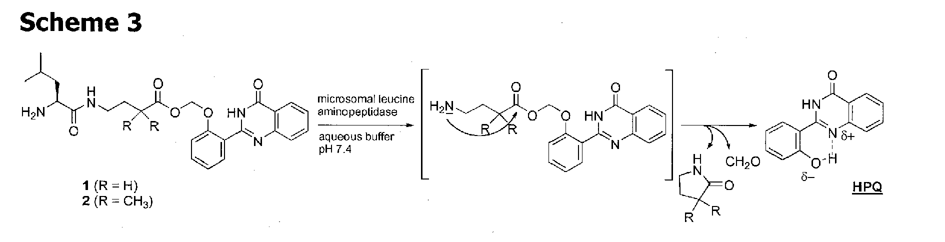
**Figure 1** (ordinate in arbitrary fluorescence units a.u.) represents the emission spectra (λ_{exc} = 365 nm) of substrate **2** (20 µM) before (curve a) and after incubation with leucyl aminopeptidase for 1 h (curve b) at 25 °C in Tris-HCl buffer. The experiment was monitored with a standard multi-well plate fluorescence spectrophotometer (Spectramax Gemini by Molecular Devices).
**Figure 2** presents a fluorogenic test (λ_{exc} = 365 nm, λₑₘ = 495 nm) at 25 °C for the activity of leucyl aminopeptidase in Tris-HCl (25 mM, pH 7.4) using 38 µM of substrate **1** or **2 ,** and 0.02 U mL⁻¹ of leucyl aminopeptidase. The following curves for time-dependent fluorescence signal evolution were obtained : (a) substrate **2** with enzyme, (b) substrate **1** with enzyme (c) substrate **2** without enzyme but with added bovine serum albumin (0.1 mg mL⁻¹), (d) substrate **1** without enzyme but with added bovine serum albumin (0.1 mg mL⁻¹)_{.}

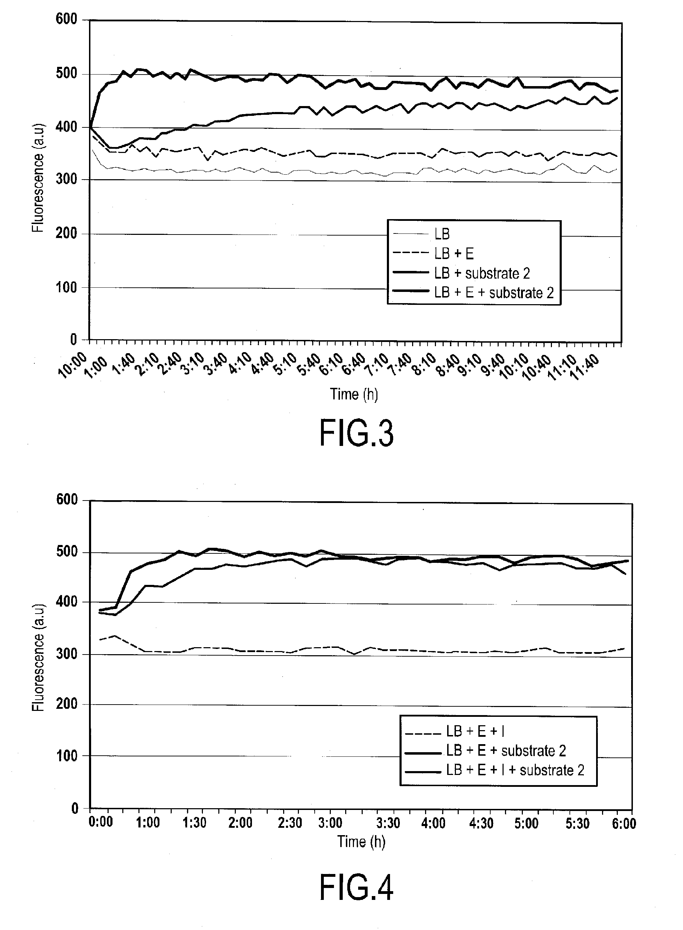

**Figure 3** presents the time-dependent signal evolution obtained with pure enzyme in complex LB (Lysogeny broth) medium (from Fisher, "LB Broth Lennox", ref 240230) rather than in pure buffer (TRIS-HCl).

### Incubation conditions :

Substrate **2** concentration : 6 µM ; enzyme : leucyle aminopeptidase (0.01 U); 37°C; LB medium ; emission wavelength : 495 nm ; Spectramax Gemini from Molecular Device.

### Interpretation of the plots :

The signal of the experiment bringing together enzyme (E) and substrate **2** reaches a plateau after approximately 1 hour (« LB+E+substrate 2 »), to be compared to the 0.5 h found in **Figure 2****,** curve a with TRIS-HCl buffer.

The signal level of pure LB medium (« LB ») is much elevated compared to TRIS-HCl buffer **(****Figure 2****,** at *t* = 0 sec. there is hardly any fluorescence to be detected).

In the absence of enzyme (« LB+substrate 2 »), the signal plateaus after about 12 hours, corresponding to **Figure 2** **,** curve c, with TRIS-HCl buffer that encompasses a timeframe of 2.77 hours.

**Figure 4** presents the time-dependent signal evolution obtained with pure enzyme in LB medium in the presence or absence of a standard aminopeptidase inhibitor (bestatine).

### Incubation conditions :

Substrate 2 concentration : 6 µM ; enzyme : leucyle aminopeptidase (0.01 U); inhibitor concentration : 10 nM ; 37°C; LB medium ; emission wavelength : 495 nm ;

### Interpretation of the plots :

Even at this very low inhibitor (I) concentration, a delay in signal evolution can be detected (« LB+E+I+substrate 2 »). It reaches the same level as the uninhibited reaction that plateaus at about 1.5 h (« LB+E+substrate 2 ») after an extra hour (2.5 h).

This observation is borne out when replacing the pure enzyme by bacterial cells.

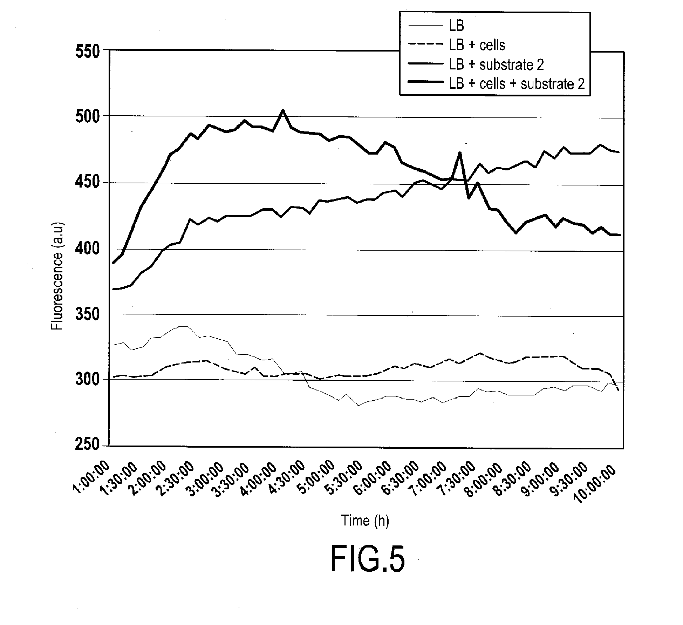

**Figure 5** depicts the results during incubation of substrate **2** with a standard bacterial cell line of E-Coli that does not overexpress a particular aminopeptidase, i.e. concentrations of aminopeptidases are at their natural, wild-type level, and accordingly very low.

### Incubation conditions :

Substrate **2** concentration : 6 µM ; bacterial cell line : DH5a (Invitrogen) ; 37°C; LB medium ; emission wavelength : 495 nm. Substrate **2** is added during the exponential growth phase at an absorbance of 0.6 to 0.8.

### Interpretation of the plots :

- The background fluorescence signal of LB medium (« LB ») is already elevated.
- Addition of bacterial cells to this medium (« LB + cells ») does not change the signal level notably.
- Substrate 2 degrades slowly under the incubation conditions to reach a signal plateau after 10 hours (« LB + substrate 2 »).
- In spite of the very low expression level of aminopeptidases in regular bacterial cell lines the incubation of substrate 2 with these cells in regular LB medium leads to a marked signal increase, reaching a plateau after 2.5 hours. This result demonstrates the high response rate of substrate **2** and has to be contrasted with the experiment illustrated in **Figures 1** and **2** that employed much more favourable conditions (high concentration of pure enzyme catalyst and 38 µM substrate concentration).

Comparable results were obtained with other E. coli cell lines : TAM1 (RapidTrans, www.activemotif.com,) and Top10 (Invitrogen).

## Claims

1. A peptidase substrate of formula (I) : 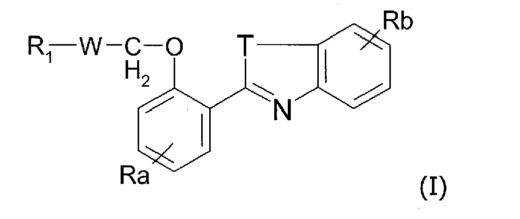 wherein
- R₁ is a peptidyl or amino acid moiety directly linked to W via its carbonyl function or one of its carbonyl functions,
or, R₁ is a chain R'₁-NH-Y-C(O)- in which R'₁ is a peptidyl or amino acid moiety directly linked to the NH of the R₁ chain via its carbonyl function or one of its carbonyl functions and Y is an organic spacer,
- W is O, NH or NR₂ with R₂ representing a (C₁-C₄)alkyl,
- T is -NH-C(O)- or -S-,
- Ra and Rb, identical or different, are, each independently, hydrogen, Cl, Br, -OCH₃, -NO₂, -NRcRd with Rc and Rd, identical or different, representing a (C₁-C₄)alkyl.

2. A peptidase substrate according to claim 1 wherein Y represents :
- CR₃R'₃-(CR₄R'₄)ₙ -CR₅R'₅-CR₆R'₆- in which n is equal to 0 or 1 and R₃, R'₃, R₄, R'₄, R₅, R'₅, R₆ and R'₆, identical or different, are, each independently, a hydrogen atom, a (C₁-C₄)alkyl optionally substituted, a aryl group optionally substituted or a lateral chain of a natural amino acid, or R₃ and R'₃ and/or R₄ and R'₄ and/or R₅ and R'₅ and/or R₆ and R'₆ are linked together to form a (C₃-C₇)cycloalkyl.

3. A peptidase substrate according to claim 2 wherein R₃, R'₃, R₄, R'₄, R₅, R'₅, R₆ and R'₆, identical or different, are, each independently, a hydrogen atom, a methyl group or a lateral chain of a natural amino acid.

4. A peptidase substrate according to claim 2 or 3 wherein R₆ and R'₆ are identical and represent a hydrogen atom or a methyl group.

5. A peptidase substrate according to any one of claims 2 to 4 wherein R₃ represents a hydrogen atom and R'₃ represents a lateral chain of a natural amino acid.

6. A peptidase substrate according to any one of claims 2 to 5 wherein R₄, R₄, R₅ and R'₅ are identical and represent a hydrogen atom.

7. A peptidase substrate according to any one of claims 1 to 6 of formula (IA) : 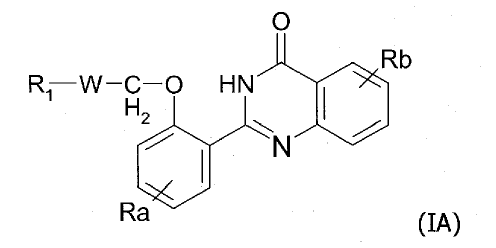 wherein R₁, W, Ra and Rb are as defined in claim 1.

8. A peptidase substrate according to any one of claims 1 to 7 wherein W is O.

9. A peptidase substrate according to any one of claims 1 to 8 wherein W is NCH₃.

10. A peptidase substrate according to any one of claims 1 to 9 wherein Ra and Rb represent a hydrogen atom.

11. A peptidase substrate according to any one of claims 1 to 10 wherein the peptidyl or amino acid moiety R₁ or R'₁ has at most 10, preferably at most 4, amino acids in which the amino acids are identical or different and preferably chosen between the natural amino acids, and the N-terminus can be substituted by an acyl group -COR', R' being a (C₁-C₆)alkyl group or a O-(C₁C₆)alkyl group.

12. A fluorescent precipitate obtainable from a peptidase substrate according to any of claim 1 to 11 by cleavage of the covalent link between W and R₁.

13. A fluorescent precipitate according to claim 13 with excitation and emission characteristics different from those of the peptidase substrate.

14. A process for detecting the presence of a catalytically active peptidase comprising the steps of :
- contacting a sample suspected of containing said peptidase with a substrate according to any one of claims 1 to 11;
- applying conditions suitable to allow formation of a fluorescent precipitate according to claim 12 or 13; and
- quantitatively or qualitatively analyzing said fluorescent precipitate.

15. A process according to claim 14 wherein the analysis of the fluorescent precipitate comprises the steps of :
- exposing the fluorescent precipitate to a light source capable of producing light at a wavelength of absorption of the fluorescent precipitate; and
- detecting the resultant fluorescence of the precipitate.
